# EUROPEAN PATENT APPLICATION

(11) **EP 4 620 508 A1**
(43) Date of publication of application: **24.09.2025**
(21) Application number: 24165190.0
(22) Date of filing: 21.03.2024
(51) Int. Cl.: A61M 39/10, A61J 1/20

(54) **CLOSED SYSTEM TRANSFER DEVICE AND ADAPTED LUER CONNECTOR**

(71) Applicant: B. Braun Melsungen AG, 34212 Melsungen (DE)
(72) Inventor: NIXON, James, Allentown, PA, 18104 (US); JANDERS, Mike, Northampton, PA, 18067 (US); VERBITSKY, Gary, Emmaus, PA, 18049 (US); AMORY, Luke, Bethlehem, PA, 18015 (US); KOPP, Florin, 26419 Schortens (DE)
(74) Representative: Winter, Brandl - Partnerschaft mbB

(57) **Abstract**

A closed system transfer device (CSTD) is described with a housing (15) and a detachable Luer connector (20) received in an axial recess (60) of the housing. The Luer connector (20) is attached to the housing (15) via at least one snap hook (68) inside the housing (15) that engages a snap ledge (70) on a central axial pin (40) of the Luer connector (20) to thereby defining a first axial coupling position (CP1) of the Luer connector (20). In this first axial coupling position (CP1) the Luer connector is allowed to freely rotate in both (CW and CCW) directions within the housing (15). The Luer connector (20) is further configured to be axially shiftable further into a second axial coupling position (CP2) in which rotation of the Luer connector (20) is prevented by form fit engagement between Luer connector (20) and housing (15).

## Description

The present disclosure relates to a closed system transfer device with an adapted Luer connector. Such Luer connector has a Luer taper which is a standardized system of small-scale fluid fittings used for making leak-free connections between a male-taper fitting and its mating female part on medical and laboratory instruments. Luer standards and testing methods are governed by ISO 80369.

### Background to the disclosure

A medical fluid line system, in particular an infusion set, is generally (not necessarily always) a tube line system with a tube, for example an infusion tube, which is flexible at least in sections and one proximal end of which (i.e. the end pointing away from the patient's body), can be connected with a coupling, for example in the form of a Luer lock, a spike/spike or similar connectors for connection, for example, to an infusion container such as an infusion bag or infusion bottle or to an infusion machine, and the other distal end (i.e. facing the patient's body) of which can be connected with a coupling (e.g. constructed according to the Luer system) for an injection catheter or similar patient access or an infusion set-integrated patient access (injection catheter) itself.

### State of the art

CMR drugs, which are used for cancer therapy, for example, primarily damage growth-intensive tumor cells during therapeutic use. Many of these drugs themselves have carcinogenic properties. To prevent contact with CMR drugs by persons not undergoing therapy, so-called "closed system transfer devices" (CSTDs) are increasingly being used in the manufacture and administration of ready-to-use preparations. An important component of CSTDs are coupling systems, also known as dry breaks, which enable the safe transfer of CMR drugs and provide a dry seal after disconnection in order to protect the environment from contamination (e.g. through leakage or droplet formation on the surfaces of the coupling partners after disconnection). A dry break coupling of this type consists of two parts, a male dry break and a female dry break. There are already various manufacturers of CSTD dry breaks on the market, for example under the names Chemfort (from Simplivia), ChemoLock (from ICU Medical), Equashield (from Equashield) or PhaSeal (from BD).

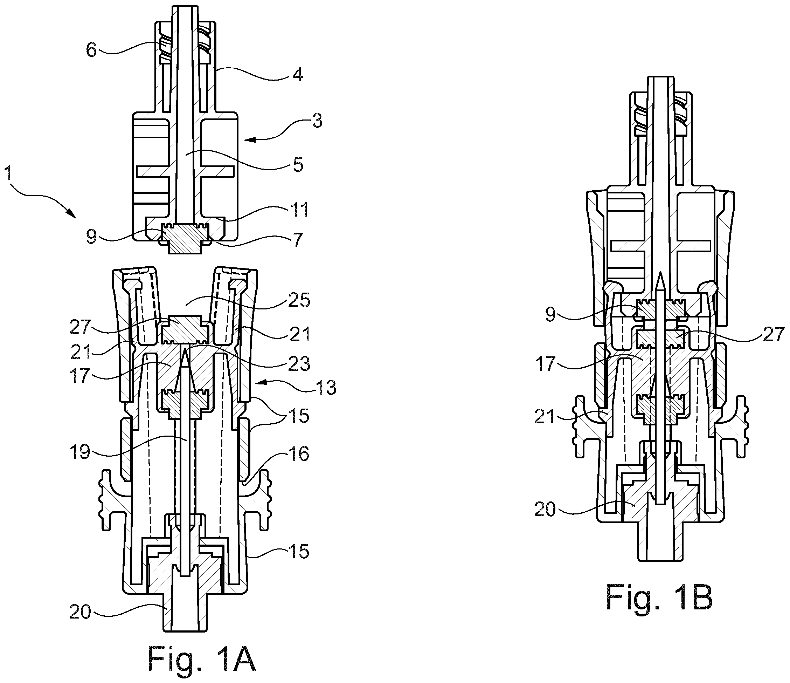

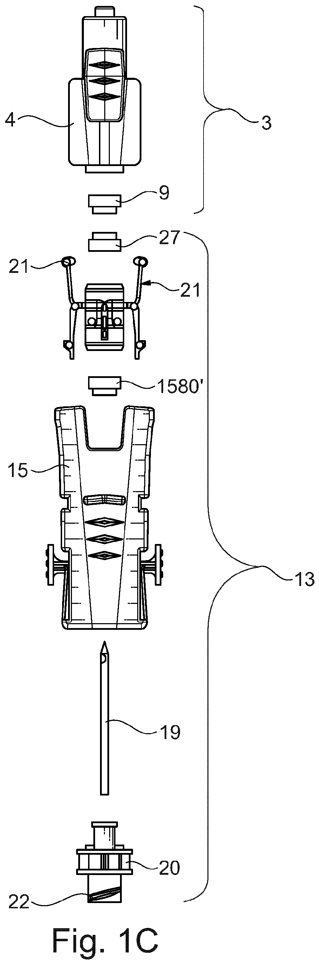

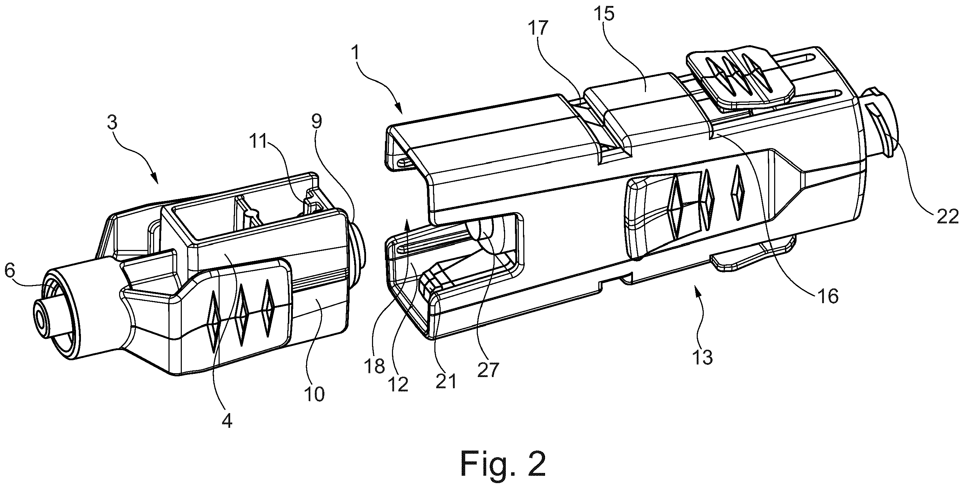

The applicant is also working on such CSTDs with dry breaks. One embodiment of such a "Vial-to-Vein Closed System Drug-transfer Device" (CSTD system), i.e. a closed/self-sealing vial-liquid removal/pass-through device, is described by way of example in the publication WO 2022 232405 A1, the disclosure of which is expressly included in the present application, and is shown in Figures 1A to 1C and 2, to which reference is already to be made here. Figures 1A and 1B show sectional views of the CSTD system in the decoupled (Figure 1A) and coupled (Figure 1B) states. Figure 2 shows a perspective view of the CSTD system. Figure 1C shows an explosion view of the CSTD system.

As shown in Figures 1A and 2, this CSTD system 1 has a first coupling adapter part 3, hereinafter referred to as a female dry break, with a housing 4 in which an axially extending through-fluid channel or a fluid channel component 5 is formed or inserted, which is closed at a proximal flange side 7 by means of a septum or membrane 9. Together with the housing 4, the fluid channel component 5 forms a (Luer lock) coupling 6 at the distal end section of the first coupling adapter part 3, with which the first coupling adapter part 3 can be connected to a medical device, for example to an entity containing a medical agent. The housing 4 or the fluid channel component 5 of the first coupling adapter part 3 also form a number of latching elements in the form of undercuts 11.

The CSTD system 1 also has a second coupling adapter part 13, referred to below as a male dry break, with a housing 15, which forms a number of latching elements in the form of undercuts 16, a slide or piston 17, which is mounted in the housing 15 so as to be relatively displaceable, a hollow needle 19, which is mounted in the housing so as to be relatively displaceable in the piston 17 and which has a retaining anchor or an anchor sleeve 20 at its proximal end section (facing away from the needle tip) and a number of elastically deformable latching arms 21 which are fixed or formed at the piston 17 and configured and designed to come into latching engagement both with the undercuts 11 of the first coupling adapter part 3 and with the undercuts 16 of the second coupling adapter part 13. As can be seen from Figure 1C and 2, the anchor sleeve 20 is designed as a female Luer connector with a thread 22.

As can be taken from Figure 1A, for the relatively displaceable mounting of the hollow needle 19 in the piston 17, the latter has a (central) through channel 23, which is closed at a distal coupling or flange side 25 of the second coupling adapter part 13 by means of a septum or membrane 27.

It can be seen from the illustration in Figure 2 that the arrangement is such that the two housing parts 4 and 15 can be pushed into one another without twisting. This is made possible by the fact that the housing 15 has a cross-sectionally rectangular receiving opening 18, in which the housing 4 of the first coupling adapter part 3 can be received with a fit, whereby ramp or alignment walls 10 of the housing 4, in cooperation with side inner walls 12 of the receiving opening 18, assume a guiding function and ensure an anti-rotation lock.

Fig. 1B shows the function of the CSTD system 1 as shown in Fig. 1A.

Accordingly, in order to establish a fluid connection between the first and second coupling adapter parts 3, 13, the membrane 27 of the second coupling adapter part 13 is pressed against the membrane 9 of the first coupling adapter part 3 in a sealing manner in a first movement time window. In a second movement time window, the housing 15 of the second coupling adapter part 13 is moved further forward in the direction of the housing 4 of the first coupling adapter part 3, whereby this further forward movement in a second movement time window causes the hollow needle 19 to pierce both adjacent membranes 9, 27, thereby establishing a fluid connection between the through-fluid channel 5 and the hollow needle 19. In this second movement time window at first the latching arms 21 engage the undercut 11. The movement of the second coupling adapter part 13 in the second movement time window ends with the engagement of the latching arms 21 in the undercut 16 of the coupling adapter parts 13. The engagement with the undercut 11 remains throughout the movement into the final position shown in Figure 1B.

As explained above, the hollow needle is held at its proximal end by the anchor sleeve 20, which at the same time also represents a connection/connector for a syringe, for example, and whose sleeve interior is in fluid connection with the fluid channel 5 of the first coupling adapter part 3 via the hollow needle 19.

Separation/disconnection of the two coupling adapter parts 3, 13 takes place in exactly the reverse order, whereby, after disengagement of the latching arms 21 by means of a pushbutton 15A , the hollow needle 19 is first pulled out of the two membranes 9, 27 by a subsequent movement of the second coupling adapter part 13 away from the first coupling adapter part 3 (i.e. the hollow needle 19 is pulled out of the two membranes 9, 27 and then (i.e. in accordance with the above second movement time window) the two membranes 9, 27 are lifted away from each other without any liquid being able to drip either from the syringe or from the vial/fluid conduit system.

An important component of such CSTDs are therefore the two coupling adapter parts (dry connections / dry breaks), which enable the safe transfer of liquid (medicinal product) and close dry after separating / disconnecting the connection in order to protect the environment from contamination (e.g. due to leakage or droplet formation on the surfaces of the coupling partners / coupling adapter parts after disconnecting the connection). These CSTD coupling adapter parts can be installed on different products (not only on one vial and one syringe). For example, the first coupling adapter part described above could also be connected via its Luer lock connection to an injection port of the Luer lock type of an infusion set in order to subsequently be able to connect a syringe with the corresponding second coupling adapter part (counter-adapter part/syringe adapter part) to it. It can then also be assumed that both coupling adapter parts of the CSTD system seal dry after disconnection and that the inner channels of both coupling adapter parts remain tightly sealed.

As explained above, the CSTD system in general needs a Luer connector 20 for connection to a fluid transfer device such as a syringe. In the coupled state, it is preferable that the syringe can freely rotate to avoid accidental disconnection.

For the above coupling purposes several configurations are known in the prior art. A syringe adaptor lock is marketed by the company Simplivia Healthcare Ltd. This syringe adaptor lock has a ratcheting connector that allows a fluid transfer device to be connected but not disconnected. This inability to disconnect could prevent clinicians from performing necessary deliveries, for instance with needles for subcutaneous injection. Moreover, this syringe adaptor has a fixed Luer connector that allows for connection and disconnection, but the fixed Luer could lead to accidental removal in normal usage, therefore exposing the clinician or patient to harmful drugs.

In WO 2017 /125920 A1 and in WO 2018/136357 A1 assigned to BECTON DICKINSON AND COMPANY LIMITED, a syringe adaptor is shown which has two axial positions. In a first axial position, the Luer connector can spin in both directions, but in the second axial position it only restricts rotation in one direction for connection, while the other direction ramps prevent disconnection. The configurations known from EP 3 725 347 A1 - owned by Equashield Medical Ltd. - have similar issues to those mentioned above with both their removable and non-removable versions.

Summing up, the current options on the market require the user to choose between the safety of free rotation and the flexibility of being able to disconnect.

It is the object underlying the present disclosure to provide a CSTD system with a female Luer connector which allows any mating fluid transfer device to be easily connected and disconnected while maintaining the safety aspect of free rotation of the Luer connector in the coupled state.

This object is achieved by a CSTD system with the features of claim 1 and with a Luer connector having the features of claims 10 and 11, respectively.

The closed system transfer device (CSTD) has a housing and a detachable Luer connector received in an axial recess of the housing for connection to a fluid transfer device such as a syringe. The Luer connector is attached to the housing via at least one snap hook inside the housing that engages a snap ledge on a central axial pin of the Luer connector. The decisive characteristic is to be seen in that when the snap connection is established, a first axial coupling position of the Luer connector is defined in which the Luer connector is allowed to freely rotate in both (CW and CCW) directions within the housing. In this stage, however, the Luer connector is also capable of limited axial movement within the housing between said first position and a second position. To this end, the Luer connector is further configured to be axially shiftable further into a second axial coupling position in which rotation of the Luer connector is prevented by form fit engagement between Luer connector and housing.

Thus, the Luer connector of the present disclosure has two axial positions, where one allows rotation and one does not, which gives clinicians a passive safety feature along with the ability to disconnect if needed. With the new concept of the present invention, the user would push the luer further into the housing to get to the second axial position where there is a form fit engagement between Luer connector and housing, and then twist to tighten or loosen the connection of the fluid transfer device to the Luer connector.

The Luer connector with the features of claims 10 and 11, respectively, is characterized by a design which is particularly easy to manufacture while providing a reliable guidance in and a wear resistant form fit engagement with the housing.

Advantageous further developments are the subject of sub-claims.

Said form fit engagement can be established between mating outer surface geometries of Luer connector and recess of the housing, e.g. between at least one flattened section of a cylindrical main body of said Luer connector and a mating portion of the axial recess of the housing, i.e. in line with the concept of mating polygons. This concept allows to provide large mating surfaces, which minimizes wear even when using soft materials, e.g. plastics, for the coupling components.

According to an advantageous modification and alternative, said axial pin projects from a cylindrical main body of the Luer connector and said form fit engagement is established by a relief-like mating interaction between a radial shoulder of the axial recess of the housing and a radial surface of the main body facing said shoulder.

One advantageous embodiment of such relief-like interaction may be performed between at least one axial protrusion or groove of a radial shoulder of the axial recess of the housing and at least one axial protrusion or recess of a radial face of the main body. It is, however, also within the scope of the present invention to define the form fit connection by any other groove and protrusion geometry on said radial shoulder of the housing and on the radial face of the Luer connector.

When said ledge on said axial pin has a ring shape, the pin can cooperate with an arbitrary number of snap hooks inside said housing to thereby improving the coupling rigidity.

In an advantageous modification, said snap hook is formed inside on an axial hollow guidance cylinder for said central axial pin. By this concept the first axial coupling position can be securely maintained.

When said at least one axial protrusion on said radial face of the main body has the form of a radially extending rib the space required for establishing the form fit engagement with the housing can be limited while minimizing the required axial movement of the Luer connector between the first and second coupling position. At the same time said radial face of the main body can be used as an abutment surface for limiting the axial movement of the Luer connector beyond said second axial coupling position.

Preferably, said radial shoulder of the axial recess in said housing forms at least two, preferably a plurality of axial protrusions between which said at least one axial protrusion of said radial face of the main body can dive in when said Luer connector is moved into its second axial coupling position. By this arrangement, the required axial shift between the first and second axial coupling positions is restricted to the height of said axial protrusions. Moreover, said radial shoulder may form a counter-abutment face for the Luer connector, whereby it is possible to remove all axial load from the protrusions. By this, the protrusions are generally arranged in a circular fashion with gaps between these protrusions. With a plurality of protrusions there is also a plurality of gaps between adjacent protrusions so that there exists a plurality of rotational positions of the Luer connector in which the gaps in the housing are aligned with the protrusions of the main body so as to allow a movement into the second axial coupling position. The more protrusions on the housing, the more rotational positions of the Luer connector allow the second coupling position and the less the user must rotate the Luer connector to bring said at least one protrusion on the Luer connector in axial alignment with a protrusion on the housing.

Designing the Luer connector as a female Luer connector provides the best concept for connecting a syringe to the CSTD system.

An advantageous embodiment of a Luer connector for use with a CSTD system according to the above described invention comprises a central main body configured to be received in a mounting recess of the housing of the CSTD system, a first hollow cylindrical pin extending from said central main body in a first axial direction and carrying a Luer lock thread, and a second hollow cylindrical pin extending from said central main body in the second axial direction and carrying a snap ledge configured to engage with at least one snap hook inside the housing in a first axial position inside said housing, wherein said second hollow cylindrical pin extends from a radial face of the central main body and said radial face carries at least one axial protrusion or recess configured to lockingly interact in a form fit manner with said housing in a second axial position further inside said housing .

According to an advantageous modification the Luer connector for use with a CSTD system according to the above described invention comprises a central main body configured to be received in a mounting recess of the housing of the CSTD system, a first hollow cylindrical pin extending from said central main body in a first axial direction and carrying a Luer lock thread, and a second hollow cylindrical pin extending from said central main body in the second axial direction and carrying a snap ledge configured to engage with at least one snap hook inside the housing in a first axial position inside said housing, wherein said second hollow cylindrical pin extends from a radial face of the central main body which has at its outer circumference at least one flattened section configured to lockingly interact in a form fit manner with a mating recess portion of said housing in a second axial position further inside said housing.

According to a further development said radial face of said central main body may carry at least one axial protrusion configured to limit the axial movement of said Luer connector into said housing beyond said first axial coupling position further inside said housing.

The weight of the Luer connector can be minimized if said central main body has the shape of a hollow cylinder with a plurality of radially extending cut-outs between its end faces.

Now, the present invention will be described with reference to the following schematic drawings illustrating embodiments of the invention in more detail. These figures show:
Figures 1A, 1B, 1C and 2 show a part of a CSTD system of known design and function;
Figures 3A and 3B show side views of a first embodiment of a CSTD system with a male dry break and a female Luer connector in a decoupled (Figure 3A) and coupled (Figure 3B) state;
Figure 4 shows a cross-sectional view of the male dry break and coupled female Luer connector in its first axial coupling position;
Figures 4A and 4B show in an enlarged scale partial sectional views of the coupled Luer connector in the first (Figure 4A) and second (Figure 4B) axial coupling positions;
Figure 5 is a perspective view of the Luer connector of Figures 3 and 4;
Figure 6 shows in an enlarged scale an axial view - when seen along arrow "VI" in Figure 4 - of the male dry break shown in Figures 3 and 4;
Figure 7 is an axial view of a modified embodiment of the male dry break;
Figure 8 is an axial view of a further modification of the CSTD system;
Figure 9 is a perspective view of a female Luer connector to be used with a male dry break shown in Figure 8; and
Figure 10 is a perspective view of a modified male dry break which can be combined with a Luer connector of Figure 9;
Figures 11A and 11B show cross sectional views of a further embodiment of the Luer connector in its first and second axial coupling positions CP1 and CP2 inside a casing of a male dry break when seen in a sectional plane XI-XI in Figure 15;
Figures 12A and 12B show cross sectional views of the Luer connector of Figure 11 in its first and second axial coupling positions CP1 and CP2 when seen in a sectional plane XII-XII in Figure 15;
Figure 13 shows the Luer connector of Figures 11 and 12 in an enlarged perspective view;
Figure 14 shows the casing of the male dry break shown in Figures 11 and 12 in a perspective view; and
Figures 15 is an enlarged front view of the casing of the male dry break shown in Figure 14 when seen along arrow "XV" in Figure 14.

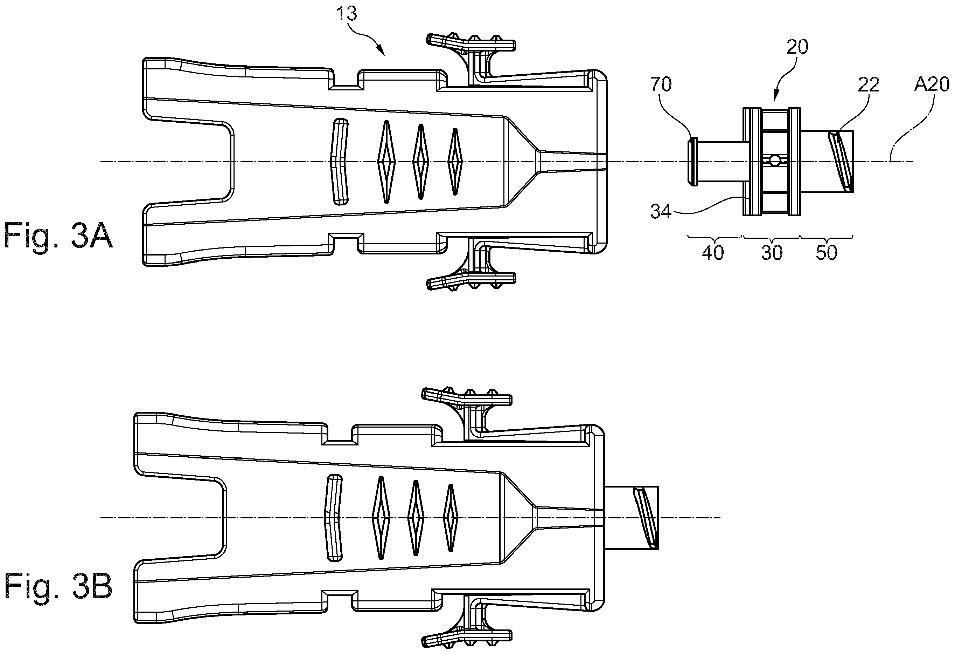

Figures 3 to 6 show a first embodiment of a closed system transfer device with a male dry break 13 and a female Luer connector 20 which can be coaxially coupled to the male dry break 13 to provide a connection to a fluid transfer device such as a syringe (not shown). The Luer connector 20 with an axis A20 has a hollow cylindrical shape with three portions: a central portion forming a substantially circular cylindrical main body 30, a hollow cylindrical axial coupling pin 40 for engagement with the male dry break 13, and a Luer connection portion 50 carrying two diametrically opposed threads 22.

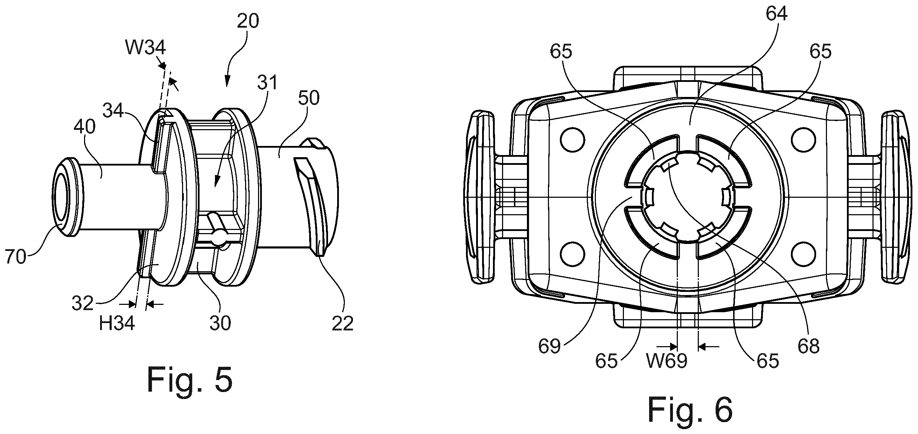

In order to save weight and material the central main body 30 has the shape of a hollow cylinder with a plurality of radially extending cut-outs 31 (see Figure 5) between its end faces.

Figure 3A shows the components in a de-coupled state and Figure 3B in the coupled state in which the coupling pin 40 is received in a stepped recess 60 of the housing 15 with an axis A15 so that only the Luer connection portion 50 projects therefrom.

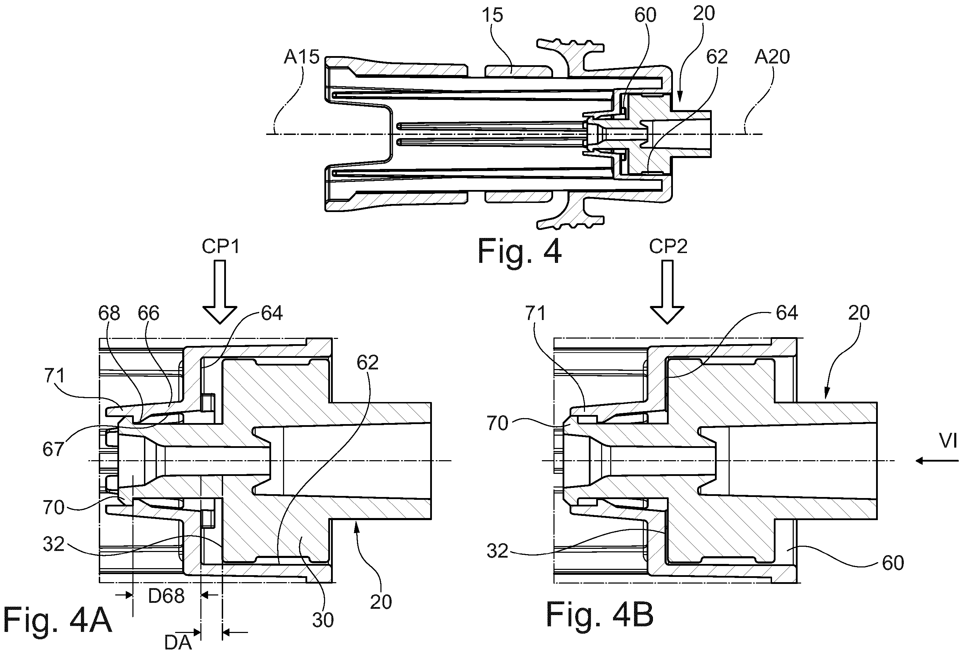

As can be best seen from Figures 4, 4A and 4B, the recess 60 has a first cylindrical recess portion 62 which extends up to a radial shoulder 64 from which a slightly conical hollow snap pin 66 extends in the axial direction away from the shoulder 64. In a specific distance D68 from the axial shoulder 64 the hollow snap pin 66 has an inwardly projecting and circumferentially extending snap hook 68 for interaction with a ring- shaped snap ledge 70 at the front end of the coupling pin 40 of the Luer connector 20. The inner wall portion 67 of the hollow snap pin 66 leading to the snap hook 68 is conical so as to guide the hollow snap pin 66 when the Luer connector 20 is coupled to the male dry break 13. The hollow snap pin 66 is radially elastic. As soon as the snap ledge 70 passes the snap hook 68, the Luer connector 20 is in its first axial coupling position CP1 shown in Figures 4 and 4A in which the Luer connector 20 is axially secured inside the housing 15 and can no longer be removed therefrom. Moreover, in this first axial coupling position CP1 the Luer connector 20 can freely rotate in both (CW and CCW) directions within the housing 15.

The dimensions of the stepped recess 60 and of the Luer connector 20 are adapted to each other such that in this first axial coupling position CP1 the cylindrical main body 30 is substantially flush with an end face 14 of the housing 15 of the male dry break 13 and a radial front surface 32 of the main body 30 is in an axial distance DA from the shoulder 64 of the recess 60.

For connecting and disconnecting any fluid transfer device, e.g. a syringe, to and from the Luer connector 20, it is desirable that the Luer connector 20 does not rotate. In this respect it is to be noted that with a mounted fluid transfer device on the Luer coupling, the Luer connection portion 50 is no longer accessible and cannot be gripped.

For solving this problem, the Luer connector 20 is further configured to be axially shiftable further along the axis A15 from the first axial coupling position CP1 into a second axial coupling position CP2 (see Figure 4B) in which any rotation of the Luer connector 20 is prevented by form fit engagement between the Luer connector 20 and the housing 15.

In more detail, and according to a first embodiment shown in figures 3 to 6, the form fit engagement is established by a relief-like mating interaction between the radial shoulder 64 of the axial recess 60 of the housing and the radial surface 32 of the main body 20 facing said shoulder 64. The main body 20 is designed as a substantially hollow cylinder with a plurality of radially extending cut-outs 31 between its end faces.

As can be best seen from Figures 5 and 6, the radial surface 32 of the main body 30 of the Luer connector 20 has a diametrically extending axial protrusion 34 in the form of a rib with a width W34 and a height H34. For providing a form fit with said axial protrusion 34 in the second axial coupling position CP2, the radial shoulder 64 of the axial recess 60 of the housing 15 has four circumferentially distanced axial protrusions 65 between which four locking grooves 67 remain. The locking grooves 69 have a width W69 adapted to the width of the axial protrusion 34 so that upon bringing the axial protrusion 34 in axial alignment with two diametrically opposite locking grooves 69 and moving the Luer connector 20 from the first axial coupling position CP1, in which the protrusions of the two parts do not engage each other and the Luer connector can freely rotate in both directions, towards the second axial coupling position CP2, the axial protrusion 34 can dive into two of the locking grooves 69. In this position the Luer connector 20 can no longer rotate, and the user or clinician can then twist to tighten or loosen the connection of the fluid transfer device to the Luer connector.

When moving the Luer connector 20 into the second axial coupling position CP2 the coupling pin 40 is concentrically guided by a hollow cylindrical extension 71 of the hollow snap pin 66, as can be seen from figures 4A and 4B. In second axial position, either the axial protrusion 34 or the end surface 32 abuts at the radial shoulder 64 of the axial recess 60.

Thus, the above embodiment the Luer connector 20 has two axial coupling positions, where one allows rotation and one does not, which gives clinicians a passive safety feature along with the ability to disconnect a fluid transfer device, if needed. In contrast to current options on the market which require the user to choose between the safety of free rotation and the flexibility of being able to disconnect, the described embodiment gives the user the option to push the Luer connector 20 further into the housing 15 to get to the second axial coupling position CP2.

It follows from the above that the axial protrusions 65 on the housing 15 are arranged in a circular fashion with gaps or locking grooves 69 in between a multitude of the protrusions 65. Since the axial protrusions 34 of the Luer connector 20 must align with the gaps in between the protrusions 65 of the housing in order for the Luer connector 20 to be able to advance to the second axial coupling position CP2, there exist four rotational positions of the Luer connector 20 in which the user can push the Luer connector 20 further into the housing 15. Therefore, the user must rotate the Luer connector 20 by a maximum of 90 degrees until he feels the Luer protrusions 34 slip between the locking grooves 69 of the housing 15, at which point the Luer connector 20 will advance to the second axial coupling position CP2 and rotation will become restricted.

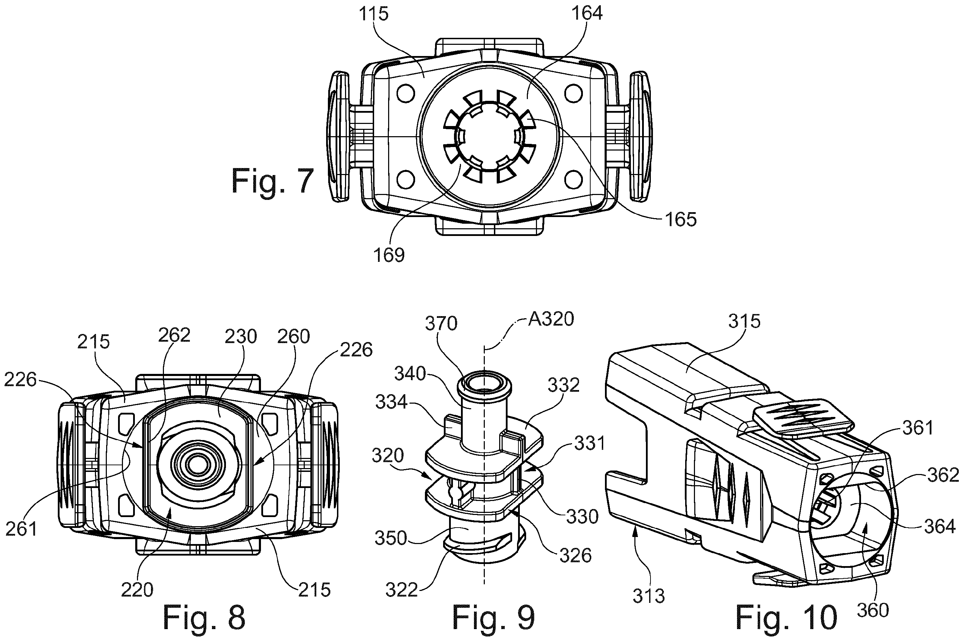

Figure 7 shows an embodiment which allows the user to reduce the rotation angle for bringing the protrusions and grooves in axial alignment. In this embodiment, the shoulder 164 has eight protrusions 165 and locking grooves 169 so that the Luer connector 20 can axially shift into the second axial coupling position CP2 in eight rotational positions. Therefore, the user must rotate the Luer connector 20 only by a maximum of 45 degrees until he feels the Luer protrusions 34 slip between the locking grooves 69 of the housing 15.

In the above-described embodiment, the form fit engagement between Luer connector 20 and housing is established by a relief-like mating interaction between the radial shoulder 64, 164 of the axial recess 60 of the housing 15 and a radial surface 32 of the Luer connector's main body 30 facing said shoulder 64, 164.

A modified concept is shown in Figure 8. Here, the form fit engagement is established between two flattened sections 226 of the cylindrical main body 230 of the Luer connector and a mating portion 262 of the axial recess 260 of the housing 215.

In a first axial coupling position CP1 the Luer connector can freely rotate in both directions in a circular portion 261 the axial recess 260. The axial depth of the axial recess cannot be seen from Figure 8. However, it can be made deep enough to function as a guidance for the rotation of the main body 230. After bringing the flattened sections 226 in axial alignment with the mating portion 262 - illustrated in Figure 8 and being adjacent to the circular portion 261 via a step in the axial recess - the Luer connector can be further axially shifted into the second first axial coupling position CP2 in which any rotation is prevented.

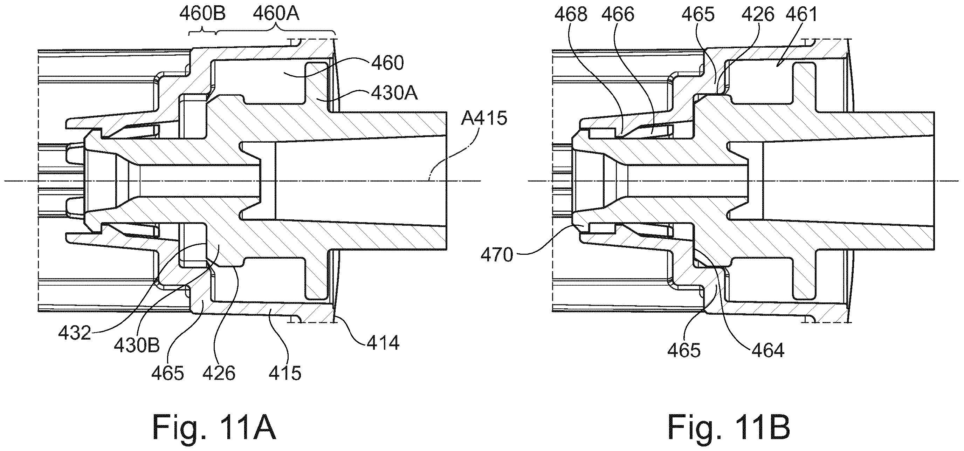

A further embodiment based on a similar concept is shown in Figures 11 to 15. For simplifying the description, elements corresponding to parts used in previously described embodiments are numbered with similar refence numerals which, however, carry a "4" as the first digit.

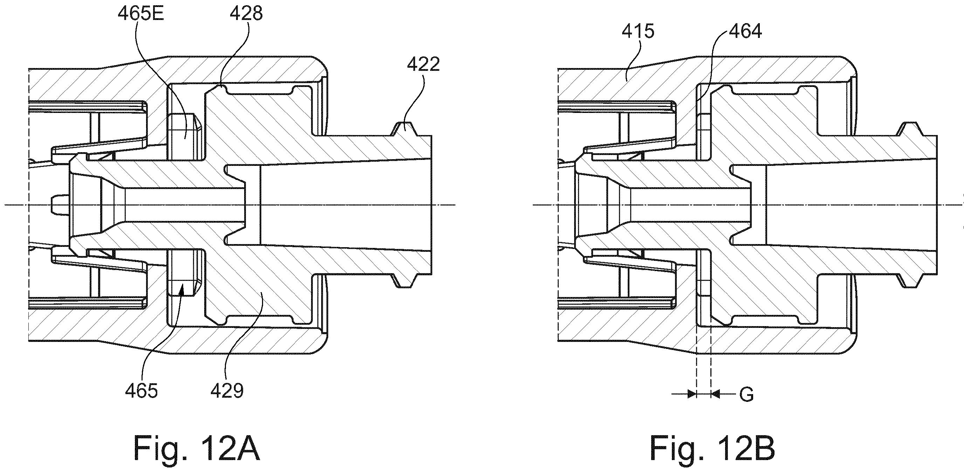

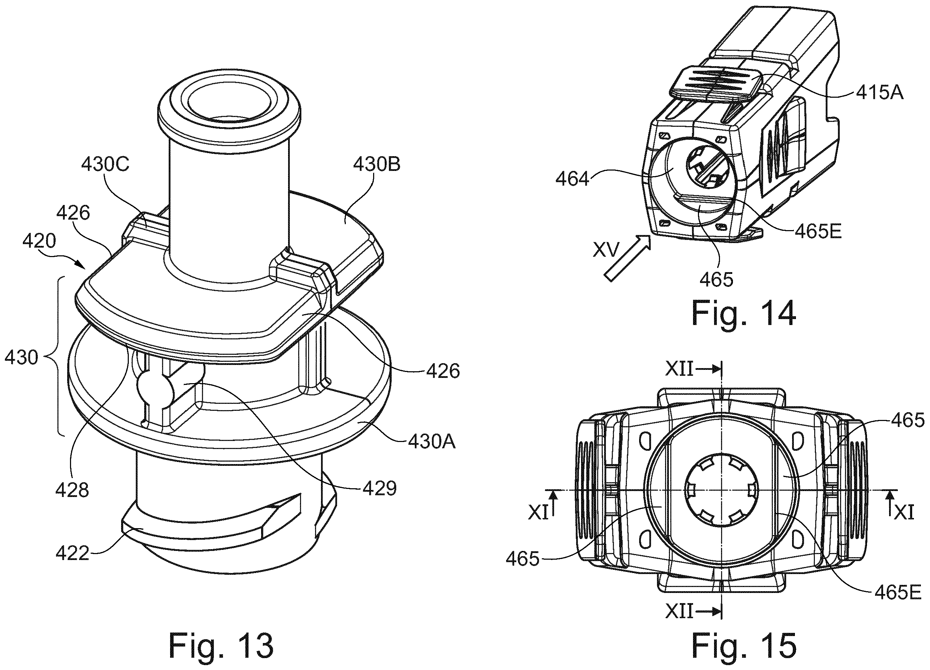

Figure 13 shows the Luer connector 420 in an enlarged perspective view, Figure 14 shows the casing 415 in a perspective view, Figures 11A and 11B show cross sectional views of the Luer connector 420 in its first and second axial coupling positions CP1 and CP2 when seen in a sectional plane XI-XI in Figure 15, and Figures 12A and 12B show cross sectional views of the Luer connector 420 in its first and second axial coupling positions CP1 and CP2 when seen in a sectional plane XII-XII in Figure 15.

As can be seen from Figures 11A, 11B and 14, the recess 460 in the housing 415 has a stepped configuration, with a circular cylindrical recess portion 461 which extends up to a radial shoulder 464 on which two diametrically opposed circle segments 465 are formed. Consequently, the cross section of the recess 460 is only circular in an area 460A. In the area 460B the cross section of the recess 460 is flattened. The configuration of the conical hollow snap pin 466 and its interaction with the snap hook 468 is the same as with the previously described embodiments.

As soon as the snap ledge 470 passes the snap hook 468, the Luer connector 420 is in its first axial coupling position CP1 shown in Figure 11A in which the Luer connector 420 is axially secured inside the housing 415 and can no longer be removed therefrom. Moreover, in this first axial coupling position CP1 the Luer connector 420 can freely rotate in both (CW and CCW) directions within the housing 415 due to its specific design as can be best seen in Figure 12.

Again, the dimensions of the stepped recess 460 and of the Luer connector 420 are adapted to each other such that in this first axial coupling position CP1 (shown in Figure 11A) the cylindrical main body 430 is substantially flush with an end face 414 of the housing 415 of the male dry break 413, and a radial front surface 432 of the main body 430 does not yet axially overlap with the circle segments 465.

As previously described with other embodiments, the Luer connector 420 is configured to be axially shiftable further along the axis A415 from the first axial coupling position CP1 into a second axial coupling position CP2 (see Figure 11B) in which any rotation of the Luer connector 420 is prevented by form fit engagement between the Luer connector 420 and the housing 415.

As shown in Figure 13, the Luer connector 420 has a main body 430 generally designed as a hollow cylinder with a plurality of radially extending cut-outs 431 between its end faces. Thus, the main body 430 has a first circular cylindrical endplate 430A adjacent to the threads 422, and a second end-plate 430B with a contour mating with the cross-section of the recess 460 in the area 460B (see Figure 11A). As shown in Figure 13, the second endplate 430B has a basically cylindrical shape with two opposing circular edges 428 and two opposing flattened edges 426. Additionally, it carries a radial rib 430C. The main body 430 is stabilized by axial ribs 429 which are preferably equally circumferentially spaced.

With this design, the Luer connector 420 in its first coupling position CP1 (shown in Figures 11A and 12A) is rotatably guided within the circular recess 460 by both endplates 430A and 430B. For securing the Luer connector 420 against rotation the flattened edges 426 of the second endplate 430B are brought into alignment with the edges 465E of the circle segments 465 so that the Luer connector 420 can be axially shifted into the second axial coupling position CP2 (shown in Figures 11B and 12B) in which the form fit engagement is established by a relief-like mating interaction between the second endplate 430B and a mating portion of the recess 460 in the area 460B. In this position the Luer connector 420 can no longer rotate, and the user or clinician can then twist to tighten or loosen the connection of the fluid transfer device to the Luer connector. Moreover, in this coupling position the radial rib 430C functions as an abutment part against the radial shoulder 464 of the housing 415 so that a gap G (see Figure 12B) remains between the radial shoulder 464 and the second endplate 430B of the main body 430. An important function of this rib 230C is to prevent over-travel of the hub into the housing so that the snap ledge of the hub 470 does not go past the ends of the snap hooks of the housing 468 and become hooked on the top edge of them.

In a modification of the described embodiment, the radial rib 430C may additionally engage a corresponding recess (not shown) in the radial shoulder 464.

Thus, the above embodiment the Luer connector 420 has two axial coupling positions, where one allows rotation and one does not, which gives clinicians a passive safety feature along with the ability to disconnect a fluid transfer device, if needed. In contrast to current options on the market which require the user to choose between the safety of free rotation and the flexibility of being able to disconnect, the described embodiment gives the user the option to push the Luer connector 420 further into the housing 515 to get to the second axial coupling position CP2.

The above-described snap connection between the Luer connector 220 of Figure 8 and the housing 215 of the male dry break can also be advantageously used with a configuration in which the Luer connector is rotationally fixed inside the housing. It is hereby declared that this modification is the subject matter of a separate invention, and the applicants reserve the right to separately claim protection for this modification. Such embodiment is shown in Figures 9 and 10. Again, elements of the system which correspond to components of the previously described embodiments are denoted with similar reference numerals which receive an additional "3" as a first digit.

The female Luer connector 320 with an axis A320 has again a hollow cylindrical shape with three portions: a central portion forming a substantially circular cylindrical main body 330, a hollow cylindrical axial coupling pin 340 for engagement with the male dry break 313 (shown in Figure 10) and providing a ring- shaped snap ledge 370, and a Luer connection portion 350 carrying two diametrically opposed threads 322.

Similar to the embodiment shown in Figure 5, the central main body 330 has the shape of a hollow cylinder with a plurality of radially extending cut-outs 331 (see Figure 9) between its end faces with flattened portions 326, thereby defining a cross-section which can be fitted into a mating axial recess 360 (shown in Figure 10) of the housing 315. The recess 360 has circular portions 361 and flattened portions 362, and when said flattened portions 326 and 362 are brought into axial alignment the Luer connector 320 can be moved into the axial recess 360 and be brought into its first axial coupling position CP1 after the ring- shaped snap ledge 370 has passed over the snap hooks (not shown) inside said housing 315.

The radial surface 332 of the main body 330 has still a diametrically extending axial protrusion 334, which, however, does no longer function as a means for locking rotation of the mounted Luer connector 320, but as a means for limiting the axial movement of Luer connector 320 into said housing 315 beyond said first axial coupling position CP1 by abutting against the shoulder 364 of the axial recess 360.

Of course, variations of the described embodiments are possible without departing from the basic idea of the invention.

The Luer connector can also be coupled to any other component of a medical fluid line system, and it can also be used elsewhere in an infusion system.

In the described embodiments the shoulder inside said axial recess of the housing is perpendicular to the axis of the housing, and the end surfaces of said Luer connector are perpendicular to the axis of the Luer connector. However, these faces may be inclined to these axes in an angle differing from 90°.

Instead of one circumferentially extending snap hook and/or a circumferentially extending snap ledge it is also feasible that a plurality of snap hooks are arranged inside the hollow snap pin and/or a plurality of circumferentially spaced snap-ledge sections are provided.

The radial elasticity of the hollow snap pin can be made more resilient by axially extending groves which are equidistantly arranged over the circumference.

Instead of protrusions on the radial face of the Luer connector it is also possible to have recesses or grooves into which protrusions on the facing shoulder interfere in the second axial coupling position CP2.

The invention thus provides a closed system transfer device (CSTD) with a housing and a detachable Luer connector received in an axial recess of the housing. Its characterizing feature is to be seen in that said Luer connector is attached to the housing via at least one snap hook inside the housing that engages a snap ledge on a central axial pin of the Luer connector to thereby define a first axial coupling position of the Luer connector in which the Luer connector is allowed to freely rotate in both (CWand CCW) directions within the housing. Said Luer connector is further configured to be axially shiftable further into a second axial coupling position in which rotation of the Luer connector is prevented by form fit engagement between Luer connector and housing.

### List of reference symbols

- 1: CSTD system
- 3: first coupling adapter part (female dry break)
- 4: housing
- 5: fluid channel component
- 6: (Luer Lock) coupling
- 7: proximal flange side
- 9: membrane
- 10: ramp or alignment walls as guide surfaces in 4
- 11: undercuts
- 12: interior side walls
- 13: second coupling adapter part (male dry break)
- 14: end face of 13
- 15: housing
- 15A: pushbutton
- A15: axis of 15
- 16: undercuts
- 17: piston
- 18: insertion opening in 15
- 19: hollow needle
- 20: anchor sleeve/Luer connector
- A20: axis of 20
- 21: latching arms
- 22: threads
- 23: (central) through channel
- 25: coupling or flange side 25 of the second coupling adapter part 13
- 27: membrane

- CP1: first axial coupling position
- CP2: second axial coupling position
- 30: main body
- 31: cut-outs
- 32: radial surface
- 34: axial protrusion
- H34: height of 34
- W34: width of 34
- 40: coupling pin
- 50: Luer connection portion
- 60: snap ledge
- 62: inner wall
- 64: shoulder
- 65: axial protrusion
- 66: hollow snap pin
- 67: inner wall
- 68: snap hook
- 69: locking grooves
- W69: width of 69
- 70: snap ledge
- 71: cylindrical extension

- 164: shoulder
- 165: protrusion
- 169: locking grooves

- 215: housing
- 226: flattened section
- 230: main body
- 260: axial recess
- 261: circular portion
- 262: mating portion

- 313: male dry break
- 315: housing
- 320: Luer connector
- A320: axis of 320
- 322: threads
- 326: flattened portions
- 330: main body
- 331: cut-outs
- 332: radial surface
- 334: axial protrusion
- 340: coupling pin
- 360: axial recess
- 361: circular portions
- 362: flattened portions
- 364: shoulder
- 370: snap ledge

- 414: end face
- 415: housing
- A415: axis of 415
- 420: Luer connector
- 422: threads
- 426: flattened edge
- 428: circular edge
- 429: axial ribs
- 430A: first endplate
- 430B: second endplate
- 430C: radial rib
- 460: recess
- 460A: circular area of 460
- 460B: flattened area of 460
- 465: circle segments
- 465E: edges
- G: gap

## Claims

1. A closed system transfer device (CSTD) with a housing (15; 115; 215; 415) and a detachable Luer connector (20) received in an axial recess (60; 260; 460) of the housing, **characterized in that** said Luer connector (20; 420) is attached to the housing (15; 115; 215; 415) via at least one snap hook (68) inside the housing (15; 115; 215; 415) that engages a snap ledge (70; 370) on a central axial pin (40; 340) of the Luer connector (20; 320) to thereby defining a first axial coupling position (CP1) of the Luer connector (20; 320) in which the Luer connector is allowed to freely rotate in both (CW and CCW) directions within the housing (15; 115; 215; 415), wherein said Luer connector (20) is further configured to be axially shiftable further into a second axial coupling position (CP2) in which rotation of the Luer connector (20) is prevented by form fit engagement between Luer connector (20) and housing (15; 115; 215; 415).

2. CSTD system according to claim 1, **characterized in that** said form fit engagement is established between a flattened section (226; 426) of a cylindrical main body (230; 430) of said Luer connector (220; 420) and a mating portion (262; 465E) of the axial recess (260; 460) of the housing (215; 415).

3. CSTD system according to claim 1, **characterized in that** said form fit engagement is established by a relief-like mating interaction between a radial shoulder (64; 164; 464) of the axial recess (60; 460) of the housing (15; 115; 415) and a radial surface (32, 34) of the main body (30) facing said shoulder (64; 164).

4. CSTD system according to claim 3, **characterized in that** said axial pin (40) projects from a cylindrical main body (30) of the Luer connector (20), and said form fit engagement is established between at least one axial protrusion (65; 165) or groove (69; 169) of a radial shoulder (64; 164) of the axial recess (60) of the housing (15; 115) and at least one axial protrusion (34) or groove of a radial surface (32) of the main body (30).

5. CSTD system according to claim 3 or 4, **characterized in that** said at least one axial protrusion (34) on said radial surface (32) of the main body (30) has the form of a radially extending rib.

6. CSTD system according to claim 5, **characterized in that** said radial shoulder (64; 164) of the axial recess (60) in said housing (15; 115) forms at least two, preferably a plurality of axial protrusions (65; 165) between which said at least one axial protrusion (34) of said radial surface (32) of the main body (30) can dive in when said Luer connector (20) is moved into its second axial coupling position (CP2).

7. CSTD system according to one of the claims 1 to 6, **characterized in that** said snap ledge (70) on said axial pin (40) has a ring shape.

8. CSTD system according to one of the claims 1 to 7, **characterized in that** said snap hook (68) is formed inside on an axial hollow snap pin (66) guiding said central axial pin (40) to the first axial coupling position (CP1).

9. CSTD system according to one of the claims 1 to 8, **characterized in that** said Luer connector (20) is designed as a female Luer connector.

10. A Luer connector (20) for use with a CSTD system according to claim 1 or 3 to 9, comprising a central main body (40) configured to be received in a mounting recess (60) of the housing (15) of the CSTD system, a first hollow cylindrical pin (50) extending from said central main body (30) in a first axial direction and carrying a Luer lock thread (22; 422), and a second hollow cylindrical pin (40 ) extending from said central main body (30) in the second axial direction and carrying a snap ledge (70; 470) configured to engage with at least one snap hook (68; 468) inside the housing (15; 415) in a first axial position (CP1) inside said housing (15; 415), wherein said second hollow cylindrical pin (40) extends from a radial face (32) of the central main body (30) and said radial face (32) carries at least one axial protrusion (34) configured to lockingly interact in a form fit manner with said housing (15) in a second axial position (CP2) further inside said housing (15).

11. A Luer connector (220; 420) for use with a CSTD system according to one of the claims 1 or 2, comprising a central main body (230; 430) configured to be received in a mounting recess (260; 460) of the housing (215; 415) of the CSTD system, a first hollow cylindrical pin (450) extending from said central main body (230; 430) in a first axial direction and carrying a Luer lock thread (422), and a second hollow cylindrical pin (440) extending from said central main body (230; 430) in the second axial direction and carrying a snap ledge (470) configured to engage with at least one snap hook inside the housing (215; 415) in a first axial position (CP1) inside said housing (215; 415), wherein said second hollow cylindrical pin (440) extends from a radial face (432) of the central main body (430) which has at its outer circumference at least one flattened section (226; 426) configured to lockingly interact in a form fit manner with a mating recess portion (262; 460, 465E) of said housing (215; 415) in a second axial position (CP2) further inside said housing (215; 415).

12. A Luer connector (320) according to claim 11, wherein said radial face (432) of said central main body (430) carries at least one axial protrusion (rib 430C) configured to limit the axial movement of said Luer connector (320) into said housing (415) beyond said second axial coupling position (CP2) further inside said housing (415).

13. A Luer connector according to one of the claims 10 to 12, wherein said central main body (30; 230; 430) has the shape of a hollow cylinder with a plurality of radially extending cut-outs (31; 431) between its end faces.
